# EUROPÄISCHE PATENTSCHRIFT

(11) **EP 2 640 695 B1**
(45) Veröffentlichungstag und Bekanntmachung des Hinweises auf die Patenterteilung: **07.01.2015**
(21) Anmeldenummer: 11782619.8
(22) Anmeldetag: 15.11.2011
(51) Int. Cl.: C07C 263/20, C07C 265/14

(54) **VERFAHREN ZUR AUFREINIGUNG VON 4,4'-METHYLENDIPHENYLDIISOCYANAT ENTHALTENDEN MISCHUNGEN**
PROCESS FOR PURIFYING MIXTURES COMPRISING 4,4'-METHYLENE DIPHENYL DIISOCYANATE
PROCÉDÉ DE PURIFICATION DE MÉLANGES CONTENANT DU DIISOCYANATE DE 4,4'-MÉTHYLÈNEDIPHÉNYLE

(30) Priorität: 17.11.2010 EP 10191560
(43) Veröffentlichungstag der Anmeldung: 25.09.2013
(73) Patentinhaber: BASF SE, 67056 Ludwigshafen (DE)
(72) Erfinder: BOCK, Michael, 67152 Ruppertsberg (DE); JACOBS, Johannes, B-2040 Antwerpen 4 (BE); THIELE, Kai, B-2040 Antwerpen 4 (BE); MERTEN, Anne-Kathrin, 01979 Lauchhammer (DE)
(86) Internationale Anmeldenummer: PCT/EP2011/070159
(87) Internationale Veröffentlichungsnummer: WO 2012/065995

(56) Entgegenhaltungen:
- DE-A1- 2 631 168
- GB-A- 798 636

## Beschreibung

Die Erfindung betrifft ein Verfahren zur Aufreinigung von 4,4'-Methylendiphenyldiisocyanat enthaltenden Mischungen umfassend die destillative Aufreinigung eines Gemisches I enthaltend 4,4'-Methylendiphenyldiisocyanat mit einem Gehalt an hydrolysierbarem Chlor nach ASTM D4663-10 von mehr als 100 ppm mittels einer Kolonne K1, wobei der gasförmige Strom bestehend aus dem Gemisch I in der Kolonne K1 mit mindestens einer flüssigen Verbindung A in Kontakt gebracht wird, welche den gleichen oder einen höheren Siedepunkt als 4,4'-Methylendiphenyldiisocyanat aufweist und welche einen Gehalt an hydrolysierbarem Chlor nach ASTM D4663-10 von höchstens 100 ppm aufweist und wobei der am Kopf der Kolonne erhaltene gasförmige Strom O enthaltend 4,4'-Methylendiphenyldiisocyanat einen Gehalt an hydrolysierbarem Chlor nach ASTM D4663-10 von höchstens 100 ppm aufweist.

Methylendiphenyldiisocyanat (MDI) ist ein wichtiges Ausgangsprodukt für die Herstellung von Polyurethanen und verwandten Polymeren, die beispielsweise in Schäumen und Beschichtungen Verwendung finden. Reines 4,4'-MDI ist eine bei Raumtemperatur feste Verbindung, die bei 38°C schmilzt.

Die säurekatalysierte Herstellung von Methylendiphenyldiisocyanat (MDI) ausgehend von Anilin und Formaldehyd ist bekannt und führt zunächst zu einem komplexen Gemisch aus Polyaminen, das anschließend mit Phosgen umgesetzt wird. Hierbei wird zunächst ein komplexes Gemisch aus zwei- und mehrkernigem MDI erhalten, welches nachfolgend als rohes Methylendiphenyldiisocyanat (Roh-MDI) bezeichnet wird. Roh-MDI besteht insbesondere aus den zweikernigen Isomeren 4,4'-MDI, 2,4'-MDI und in geringerem Maße 2,2'-MDI (zusammen nachfolgend als rohes zweikerniges MDI bezeichnet) sowie aus drei- oder mehrkernigem MDI, welches nachfolgend als polymeres MDI bezeichnet wird (PMDI).

In bekannten Verfahren wird rohes MDI in eine PMDI-reiche Mischung und in rohes zweikerniges MDI aufgetrennt. Anschließend erfolgt üblicherweise die Abtrennung von 4,4'-MDI einerseits und einer 2,4'-MDI-reichen Mischung andererseits aus dem rohen zweikernigen MDI. Entsprechende Verfahren werden beispielsweise in den Offenlegungsschriften DE 1923214, DE 102005004170, DE 102005055189, CN 101003497 und DE 10333929 beschrieben.

Für die weitere Verwendung in den genannten polymeren Systemen ist in einigen Fällen eine hohe Reinheit, insbesondere eine hohe Isomerenreinheit notwendig, da oft nur hochgradig lineare Polymere aus 4,4'-MDI die gewünschten Endeigenschaften aufweisen. In anderen Fällen finden Mischungen der oben genannten Isomere in An- oder Abwesenheit von mehrkernigem MDI Verwendung.

Vor der weiteren Verarbeitung müssen die so hergestellten, in flüssiger Form vorliegenden Methylendiphenyldiisocyanat-Produkte temporär aufbewahrt und/oder gelagert werden. Methylendiphenyldiisocyanat (MDI), insbesondere zweikerniges MDI, bildet in flüssiger Phase nach einiger Zeit, d. h. während der Lagerung, dimere Folgeprodukte. Hierbei spielt insbesondere die Bildung von Uretdionen durch 4-Ringbildung infolge Dimerisierung zweier Isocynatgruppen und die Bildung von Uretoniminen durch 4-Ringbildung aus einer Carbodiimidgruppe und einer Isocyanatgruppe eine wichtige Rolle. Die Bildung der 4-Ringe ist grundsätzlich eine Gleichgewichtsreaktion, die durch Temperaturerhöhung auf die Seite der Isocyanate bzw. Carbodiimide verschoben werden kann. Die Bildung von Uretdionen erfolgt bei aromatischen Isocyanaten auch unkatalysiert. Eine Trimerisierung zu den sogenannten Isocyanuraten (1,3,5-Triazin-2,4,6-trione) ist ebenfalls möglich, verläuft jedoch im Allgemeinen nur bei Zugabe eines geeigneten Katalysators in nennenswerter Geschwindigkeit.

Die Bildung der in dem Methylendiphenyldiisocyanat unlöslichen dimeren Folgeprodukte führt zu nachteiligen Trübungen und Sedimentationen und resultiert in Qualitätsminderungen in der nachfolgenden Weiterverarbeitung, insbesondere durch Verstopfung von Leitungen, Apparaten und Maschinen.

Ein weiteres Problem sind in MDI enthaltene aromatische Halogenverbindungen. Bei der mit Salzsäure katalysierten Kondensation von Formaldehyd und Anilin bilden sich chlorhaltige Nebenprodukte, welche zunächst nicht abgetrennt werden, sondern weiter mit Phosgen umgesetzt werden. Bei der Reaktion der komplexen Polyamin-haltigen Mischung mit Phosgen bilden sich weitere chlorhaltige Verbindungen, insbesondere N,N-disubstituierte (sekundäre) Carbaminsäurechloride und chlorierte Phenylisocyanate.

Aromatische Halogenverbindungen sind insbesondere dann zu vermeiden, wenn sie bei erhöhten Temperaturen chemisch in Verbindungen mit leicht hydrolysierbarem Halogen umgewandelt werden. Hydrolysierbare Halogenverbindungen, insbesondere wenn sie in veränderlichen Konzentrationen auftreten, stören die Umsetzung von Isocyanaten mit Polyolen zu Polyurethanen, da durch die Halogenverbindungen die Reaktionsgeschwindigkeit beeinflusst wird. Außerdem verursachen sie eine raschere Gelbfärbung der zunächst wasserklar und farblos anfallenden Isocyanate. Aus einer Vielzahl von derartigen aromatischen Halogenverbindungen seien beispielhaft genannt: N,N-Dimethylanilinhydrochlorid, N-Chloroformylanilin, N-Methyl-N-chloroformylanilin sowie Verbindungen der Formeln 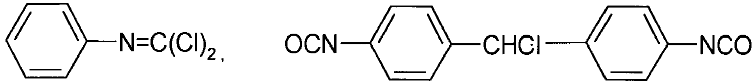 und

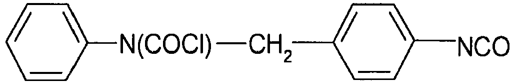

Ein Verfahren, welches den Gehalt aromatischer Halogenverbindungen in Mischungen enthaltend 4,4'-MDI reduziert oder eine solche Mischung mit geringem Gehalt an aromatischen Halogenverbindungen zur Verfügung stellt, ist somit wünschenswert.

Verfahren zur Herstellung von Methylendiphenyldiisocyanat mit geringem Gehalt an Chlorverbindungen sind aus dem Stand der Technik an sich bekannt.

Die DE-OS 2631168 beschreibt die Herstellung von bezüglich ihres Chlorgehaltes einstellbaren Diisocyanaten. Hierzu wird ein im Wesentlichen aus 2,4'- und 4,4'-MDI bestehendes Isomerengemisch zunächst in einer Destillationskolonne von der Hauptmenge der höher als 4,4'-MDI siedenden Verunreinigungen befreit und anschließend das hierbei erhaltene Destillat von den leichter als 2,4'-MDI siedenden Verunreinigungen destillativ befreit. Die vorgeschlagene technische Lösung ist jedoch apparativ sehr aufwendig. Die Abreicherung des erhaltenen 4,4'-MDI an sekundären Carbaminsäurechloriden ist zudem oft unzureichend.

DE-OS 2933601 beschreibt ein Verfahren zur Herstellung von polymerem MDI und monomerem MDI mit einem geringen Anteil an Uretdionen und hydrolysierbaren Chlorverbindungen. In einer ersten Stufe wird zweikerniges MDI von PMDI abgetrennt (Dünnschichtverdampfer 175-210°C). Das Destillat aus dem Dünnschichtverdampfer wird in Gegenwart eines Inertgases kondensiert und dann die MDI-Isomere destillativ voneinander getrennt. Das so erhaltene 4,4'-MDI enthält jedoch noch unerwünschte Verbindungen, die höher als 4,4'-MDI sieden. Das Verfahren lässt sich zudem nicht immer in ökonomischer Weise in einen Gesamtprozess integrieren.

DD-P 288599 A5 beschreibt ein Verfahren zur Reduzierung des Gehalts von chlorhaltigen Verbindungen in Isocyanaten durch Versetzen mit Carbodiimiden und anschließendes Strippen. Die thermische Enthalogenierung führt jedoch nicht zu einem vollständigen Abbau der Halogenverbindungen. So können die sekundären Carbaminsäurechloride nicht vollständig entfernt werden. Durch die hohe thermische Belastung des erhaltenen Produkts bilden sich zudem unerwünschte Abbauprodukte. Die Zugabe von Carbodiimiden bewirkt neben der angegebenen Chlorreduzierung eine Erhöhung des Molekulargewichtes durch Trimisierungsreaktionen.

Bestimmte vorwiegend schwer hydrolysierbare aromatische Halogenverbindungen, die einen höheren Siedepunkt als 4,4-MDI aufweisen, lassen sich jedoch mit den Verfahren nach dem Stand der Technik nicht oder nicht in ausreichendem Maße aus 4,4'-MDI enthaltenden Mischungen entfernen. Darüber hinaus sind die aus dem Stand der Technik bekannten Verfahren nicht immer in zufriedenstellendem Ausmaß in bekannte Verfahren zur Herstellung von MDI zu integrieren.

Es war daher Aufgabe der Erfindung ein Verfahren zur Aufreinigung von 4,4'-MDI enthaltenden Mischungen aufzufinden, das die vorgenannten Nachteile nicht oder in geringerem Umfang aufweist.
Aufgabe der vorliegenden Erfindung war es insbesondere, Mischungen aus MDI-Isomeren, insbesondere Mischungen aus 2,4'- und 4,4'-MDI sowie reines 4,4'-MDI mit einem geringen Gehalt an Uretdionen und Uretoniminen sowie hydrolysierbaren Chlorverbindungen herzustellen. Das Verfahren sollte mit einem geringen apparativen Aufwand zu realisieren sein und gegenüber MDI schonend sein.

Die Aufgabe bestand insbesondere darin, ein Gemisch enthaltend 4,4'-MDI zur Verfügung zu stellen, welches einen geringen Gehalt an hydrolysierbaren Chlorverbindungen aufweist. Insbesondere der Gehalt an chlorierten Phenylisocyanaten und an chlorierten Nebenprodukten, die aus nicht abgetrennten Nebenprodukten der Anilin-Formaldehyd Kondensation in die Phosgenierung gelangen, sollte möglichst gering sein.

Das Verfahren sollte mit möglichst geringem verfahrenstechnischem Aufwand in bestehende Technologien zur Herstellung von zweikernigem MDI zu integrieren sein.

Die vorgenannten Aufgaben werden gelöst durch das erfindungsgemäße Verfahren. Bevorzugte Ausführungsformen sind den Ansprüchen und der nachfolgenden Beschreibung zu entnehmen. Kombinationen bevorzugter Ausführungsformen verlassen den Rahmen der vorliegenden Erfindung nicht.

Das erfindungsgemäße Verfahren zur Aufreinigung von 4,4'-Methylendiphenyldiisocyanat enthaltenden Mischungen umfasst die destillative Aufreinigung eines Gemisches I enthaltend 4,4'-Methylendiphenyldiisocyanat, das einen Gehalt an hydrolysierbarem Chlor nach ASTM D4663-10 von mehr als 100 ppm, vorzugsweise mehr als 150 ppm, aufweist, mittels einer Kolonne K1, wobei der gasförmige Strom bestehend aus dem Gemisch I in der Kolonne K1 mit mindestens einer flüssigen Verbindung A in Kontakt gebracht wird, welche den gleichen oder einen höheren Siedepunkt als 4,4'-Methylendiphenyldiisocyanat aufweist und welche einen Gehalt an hydrolysierbarem Chlor nach ASTM D4663-10 von höchstens 100 ppm, vorzugsweise höchstens 50 ppm, aufweist und wobei der am Kopf der Kolonne erhaltene gasförmige Strom O enthaltend 4,4'-Methylendiphenyldiisocyanat einen Gehalt an hydrolysierbarem Chlor nach ASTM D4663-10 von höchstens 100 ppm, vorzugsweise höchstens 50 ppm, aufweist.

Der Begriff "Aufreinigung von 4,4'-Methylendiphenyldiisocyanat enthaltenden Mischungen" kennzeichnet die Umwandlung eines ersten Gemisches enthaltend 4,4'-Methylendiphenyldiisocyanat in ein zweites Gemisch, dessen Gehalt (in Gew.-%) an 4,4'-Methylendiphenyldiisocyanat höher ist als der des ersten Gemisches. Die destillative Aufreinigung umfasst jedwede ein- oder mehrstufige Aufreinigung, die in mindestens einem Schritt eine Destillation umfasst.

Der Begriff "Destillation" beziehungsweise "destillativ" kennzeichnet die Aufreinigung eines Gemisches mittels eines destillativen Trennverfahrens. Destillative Trennerfahren sind dadurch gekennzeichnet, dass die Trennwirkung auf der unterschiedlichen Zusammensetzung der siedenden Flüssigkeit und des gasförmigen Dampfes beruht.

Der Gehalt in ppm bezieht sich im Rahmen der vorliegenden Erfindung grundsätzlich auf Gewichtsanteile bezogen auf das Gesamtgewicht eines Gemisches.

Aromatische Halogenverbindungen sind chemische Verbindungen, die mindestens einen aromatischen Ring und mindestens ein Halogenatom enthalten.

Eine Kolonne ist eine Vorrichtung zur destillativen Aufreinigung eines Gemisches. Im Rahmen der vorliegenden Erfindung wird unter Kolonne eine Rektifikationskolonne verstanden. Kolonnen sind dem Fachmann an sich bekannt.

Eine Kolonne umfasst einen vorzugsweise längsförmigen Behälter mit Trennelementen. Trennelemente sind Einbauten, die die Wärme- und Stoffübertragung intensivieren. Die Kolonne umfasst zudem einen Bereich unterhalb des untersten Trennelementes, der Kondensat aufnehmen kann (den Sumpf) und einen Bereich oberhalb des obersten Trennelementes, den Kopf. Zwecks Verdampfung des zu trennenden Stoffgemisches kann unterhalb des Sumpfes der Rektifikationskolonne ein Verdampfer angeordnet sein. Zwecks Kondensation des am Kopf austretenden gasförmigen Stromes kann hinter dem Kolonnenkopf ein Kondensator angeschlossen sein.

Je nach Art der verwendeten Trennelemente wird unterschieden zwischen Boden-, Füllkörper- und Packungskolonnen. Durch einen Zulauf, der häufig am Boden einer Kolonne angebracht ist, wird das verdampfte Gemisch der zu trennenden Stoffe eingespeist. Zum Kopf hin reichert sich die leichter siedende Komponente an und kann dort abgezogen werden, während die schwerer siedende Komponente zurückgeführt wird. Im Sumpf reichert sich die schwerer siedende Komponente an und kann dort abgenommen werden.

Man unterscheidet üblicherweise drei Typen von Trennelementen. In Bodenkolonnen sind Sieb- , Glocken- oder Ventilböden eingebaut, auf denen die Flüssigkeit steht. Durch spezielle Schlitze oder Löcher wird der Dampf in die Flüssigkeit eingeperlt, so dass eine Sprudelschicht entsteht. Auf jedem dieser Böden stellt sich ein neues temperaturabhängiges Gleichgewicht zwischen der Flüssig- und Gasphase ein.

Füllkörperkolonnen können mit unterschiedlichen Füllkörpern gefüllt werden, die eine gute Verteilung der Flüssigkeit und Verwirbelung der Gasströmung bewirken. Durch die Oberflächenvergrößerung werden Wärme- und Stoffaustausch optimiert und die Trennfähigkeit der Kolonne somit erhöht. Bekannte Beispiele sind der Raschig-Ring (ein Hohlzylinder), Pall-Ring, Hiflow-Ring, Intalox-Sattel, Berl-Sattel und Igel. Die Füllkörper können geordnet, aber auch regellos (als Schüttung) in die Kolonne eingebracht werden.

Packungskolonnen mit Packungen als Trennkörper (Packungselemente) sind eine Weiterentwicklung der geordneten Füllkörper. Sie weisen eine regelmäßig geformte Struktur auf. Dadurch ist es bei Packungen möglich, Einengungen für die Gasströmung (mit erheblichem Einfluss auf den Druckverlust) zu reduzieren. Es gibt verschiedene Ausführungen von Packungen, z. B. Gewebe- oder Blechpackungen.

Die Bestimmung des Gehaltes an hydrolysierbarem Chlor erfolgt im Rahmen der vorliegenden Erfindung grundsätzlich gemäß ASTM D4663-10 und kennzeichnet den Gehalt an aromatischen Chlorverbindungen, die unter den Bedingungen der ASTM D4663-10 hydrolysierbar sind (häufig als "DHC" oder difficultly hydrolyzable chlorine bezeichnet). Davon zu unterscheiden ist der Gesamtchlorgehalt gemäß ASTM D 4661-09, welcher auch ringsubstituierte Chlorverbindungen wie Monochlorbenzol erfasst, und der sogenannte Gehalt an leicht hydrolysierbarem Chlor (EHC - "easily hydrolysable chlorine") gemäß ASTM D 5629-05, welcher die Acidität in Form von HCl kennzeichnet.

Bevorzugte Ausführungsformen des erfindungsgemäßen Verfahrens werden nachfolgend erläutert.

Die Herstellung geeigneter Ausgangsmischungen enthaltend 4,4'-Methylendiphenyldiisocyanat ist dem Fachmann bekannt.

Das Verfahren kann grundsätzlich bei jeder 4,4'-Methylendiphenyldiisocyanat enthaltenden Mischung eingesetzt werden, sofern die Mischung der destillativen Aufbereitung zugänglich ist. Der Gehalt des eingesetzten Gemisches I an der Verbindung 4,4'-Methylendiphenyldiisocyanat beträgt jedoch bevorzugt mindestens 95 Gew.-%, insbesondere mindestens 98 Gew.-%. Das erfindungsgemäße Verfahren kann grundsätzlich aber auch für Mischungen von 2,4'-MDI und 4,4'-MDI angewandt werden, wenn bei der Isomerentrennung 4,4'-MDI nicht getrennt gewonnen wird.

Dem Fachmann ist bekannt, wie er die vorgenannten Gemische enthaltend 4,4'-Methylendiphenyldiisocyanat erhalten kann. Zunächst erfolgt in einem ersten Schritt die Kondensation von Anilin und Formaldehyd und anschließend die Phosgennierung der resultierenden Polyaminmischung (Polyaminopolyphenylpolymethane).

Die Kondensation von Anilin und Formaldehyd sowie die Phosgenierung der Polyaminopolyphenylpolymethane ist aus dem Stand der Technik hinlänglich bekannt. Nach der Phosgenierung von Polyaminopolyphenylpolymethanen wird zunächst Phosgen vollständig entfernt. Dann werden die mehrkernigen Homologe des Methylendiphenyldiisocyanats (PMDI) abgetrennt, wobei sogenanntes rohes zweikerniges MDI gewonnen wird. Rohes zweikerniges Methylendiphenyldiisocyanat (MDI) ist dem Fachmann als Gemisch bekannt, welches neben 4,4'-Methylendiphenyldiisocyanat noch mindestens eines der Isomeren 2,2'- und 2,4'-Methylendiphenyldiisocyanat enthält. Der Gehalt an 4,4'-Methylendiphenyldiisocyanat in rohem zweikernigem Methylendiphenyldiisocyanat beträgt üblicherweise weniger als 80 Gew.-%, insbesondere weniger als 60 Gew.-%.

Aus rohem zweikernigem Methylendiphenyldiisocyanat wird anschließend reines 4,4'-Methylendiphenyldiisocyanat abgetrennt. Für die Abtrennung sind verschiedene Verfahren auf Basis einer Destillation oder Kristallisation oder einer Kombination aus Destillation und Kristallisation aus dem Stand der Technik bekannt.

Das erfindungsgemäße Verfahren kann vorteilhaft in bekannte Verfahren zur Herstellung von 4,4'-MDi enthaltenden Mischungen integriert werden, insbesondere indem es mit einer Destillation zur Abtrennung der genannten isomeren (zweikernigen) Methylendiphenyldiisocyanate kombiniert wird. Dies wird weiter unten näher ausgeführt.

Gemäß der vorliegenden Erfindung wird der gasförmige Strom bestehend aus dem Gemisch I in der Kolonne K1 mit mindestens einer flüssigen Verbindung A in Kontakt gebracht, welche den gleichen oder einen höheren Siedepunkt als 4,4'-Methylendiphenyldiisocyanat aufweist und welche einen Gehalt an hydrolysierbarem Chlor nach ASTM D4663-10 von höchstens 100 ppm aufweist, vorzugweise höchstens 50 ppm, besonders bevorzugt höchstens 30 ppm, insbesondere höchstens 20 ppm, ganz besonders bevorzugt höchstens 10 ppm.

Grundsätzlich kommen als flüssige Verbindungen A Verbindungen in Betracht, die entweder inert gegenüber 4,4'-Methylendiphenyldiisocyanat sind oder 4,4'-Methylendiphenyldiisocyanat selbst..

Geeignete inerte Verbindungen A sind insbesondere Dibenzylether, Terphenyl, höhere Phtalsäureester und Naphthalinderivate. Selbstverständlich kommen auch Mischungen der vorgenannten inerten Verbindungen in Betracht.

Besonders bevorzugt ist die Verbindung A jedoch 4,4'-Methylendiphenyldiisocyanat. Besonders bevorzugt wird als Verbindung A dabei 4,4'-Methylendiphenyldiisocyanat mit einer Reinheit von mindestens 97 Gew.-%, besonders bevorzugt mindestens 98 Gew.-%, insbesondere mindestens 98,5 Gew.-% verwendet. Grundsätzlich kann jedoch auch eine Mischung aus 4,4'-Methylendiphenyldiisocyanat der genannten Reinheit mit mindestens einer oben genannten inerten Verbindungen verwendet werden. In jedem Fall beträgt der Gehalt an hydrolysierbarem Chlor nach ASTM D4663-10 in der Verbindung A höchstens 100 ppm.

Ganz besonders bevorzugt ist die Verbindung A 4,4'-Methylendiphenyldiisocyanat, welches die erfindungsgemäße destillative Aufreinigung bereits durchlaufen hat. Hierdurch wird 4,4'-Methylendiphenyldiisocyanat, welches die erfindungsgemäße destillative Aufreinigung bereits durchlaufen hat und in geeigneter Weise aufbewahrt werden muss, wobei sich die eingangs erwähnten dimeren Folgeprodukte bilden, anteilig erneut dem Prozess zugeführt. Hierdurch können gleichzeitig zwei Vorteile realisiert werden: einerseits wird erfindungsgemäß 4,4'-MDI, das arm an aromatischen Halogenverbindungen ist, zugänglich gemacht und andererseits wird gelagertes 4,4-MDI, das infolge der Lagerung teilweise zu Folgeprodukten abgebaut wurde, wieder rezykliert und gelangt in hochreiner Form in die Lagerung, wodurch die Qualität des gelagerten 4,4'-MDI insgesamt optimiert werden kann. Hierbei beträgt das Lagerungs-Rücklaufverhältnis (zurückgeführte Menge an 4,4'-MDI im Verhältnis zur der Lagerung neu zugeführten (hinzugewonnenen) Menge 4,4'-MDI bevorzugt von 0,05 bis 0,4, insbesondere von 0,1 bis 0,3.

Ganz besonders bevorzugt ist die Verbindung A 4,4'-Methylendiphenyldiisocyanat, das aus einer Vorrichtung zur Lagerung von 4,4'-Methylendiphenyldiisocyanat zurückgeführt wird. Eine Vorrichtung zur Lagerung ist dabei jede Vorrichtung, die zur temporären Aufnahme des zu lagernden Stoffes oder Stoffgemisches vorgesehen ist, beispielsweise ein Behältnis, insbesondere ein Lagertank.

Die Zugabe der flüssigen Verbindung A erfolgt vorzugsweise oberhalb des obersten Trennelementes der Kolonne K1. In einer bevorzugten Ausführungsform ist die Kolonne K1 eine Packungskolonne. Die spezifische Oberfläche der Packung beträgt dabei vorzugsweise von 100 bis 1000 m²/m³, besonders bevorzugt von 150 bis 800 m²/m³, insbesondere von 200 bis 750 m²/m³, ganz besonders bevorzugt von 250 bis 600 m²/m³.

Grundsätzlich sind solche Packungen bevorzugt, die einen geringen Druckverlust verursachen. Geeignete Packungen sind insbesondere Gewebepackungen, Blechpackungen und strukturierte Packungen. Gewebepackungen sind besonders bevorzugt.

Die Zugabe der flüssigen Verbindung A erfolgt dabei vorzugsweise oberhalb des höchsten Packungselementes der Kolonne K1.

Gemäß der vorliegenden Erfindung wird der gasförmige Strom bestehend aus dem Gemisch I in der Kolonne K1 mit mindestens einer flüssigen Verbindung A in Kontakt gebracht. Grundsätzlich kommen mehrere Methoden des Inkontaktbringens in Betracht. Bevorzugt sind dabei solche Methoden, die zu einem intensiven Kontakt des Gasstromes und der flüssigen Verbindung A führen. Hierfür muss die Flüssigkeit A in geeigneter Weise verteilt werden. Entsprechende Methoden sind dem Fachmann an sich bekannt.

Die Wirksamkeit des Inkontaktbringens ist insbesondere von einer gleichmäßigen, flächendeckenden Flüssigkeitsaufgabe abhängig. Flüssigkeitsverteiler gewährleisten eine weitgehend homogene Flüssigkeitsverteilung über dem Kolonnenquerschnitt und sind dem Fachmann bekannt.

Eine Vorverteilung der Flüssigkeit kann durch ein oder mehrere Zulaufrohre oder Verteilerrinnen mit mehreren Ausflussöffnungen, die sich an der Unterseite befinden, realisiert werden.

Eine wichtige Auslegungsgröße ist die Anzahl von Aufgabestellen bezogen auf den Kolonnenquerschnitt (= Tropfstellendichte). Folgende Bauarten von Flüssigkeitsverteilern kommen in Betracht: Verteilerböden, Rinnenverteiler, Rohrverteiler und Düsenverteiler. Folgende Prinzipien der Flüssigkeitsverteilung kommen in Betracht: Stauhöhenverteilung über Bohrungen im Boden von Verteilern oder seitlich gebohrte Aufgaberöhrchen, Überlaufverteilung z.B. über seitliche Schlitze oder Überlauftüllen und Düsen.

Geeignete Flüssigkeitsverteiler sind insbesondere Kastenrinnenverteiler. Es ist vorteilhaft, wenn das Inkontaktbringen im Gegenstrom zum gasförmigen Strom I erfolgt. Hierdurch ergibt sich ein besonders niedriger Gehalt an aromatischen Halogenverbindungen im resultierenden Gemisch.

Die Zuführung des Gemisches I in die Kolonne K1 kann grundsätzlich auf verschiedene Weise erfolgen, und zwar in flüssiger Form oder in gasförmiger Form. Bei einer Zuführung in flüssiger Form ist die Verwendung eines geeigneten Verdampfers vorteilhaft. Vorzugsweise erfolgt die Zuführung des Gemisches I in die Kolonne K1 in gasförmiger Form.

Der absolute Druck im Kopf der Kolonne K1 beträgt bevorzugt höchstens 50 mbar, besonders bevorzugt von 1 bis 30 mbar, insbesondere von 2 bis 20 mbar. Die Druckdifferenz zwischen Kopf und Sumpf (Druckabfall) der Kolonne K1 beträgt vorzugsweise von 0,5 bis 30 mbar, bevorzugt von 0,5 bis 20 mbar, besonders bevorzugt von 1 bis 10 mbar, insbesondere von 2 bis 5 mbar. Ein geringer Druckabfall bewirkt eine geringere thermische Belastung des Produkts durch niedrigere Sumpftemperaturen.

Die Temperatur im Sumpf der Kolonne K1 beträgt von 140 bis 270°C, bevorzugt von 150 °C bis 240°C, besonders bevorzugt von 170 bis 230°C, insbesondere von 190 bis 230°C, ganz besonders bevorzugt von 200 bis 225 °C. Hierdurch wird die thermische Belastung bei gegebener Effizienz der Aufreinigung minimiert.

Das Rücklaufverhältnis, hier definiert als das Verhältnis von Waschflüssigkeit zu Brüden W:B [w/w] beträgt W:B = 0,01 bis 2,0 bevorzugt W:B= 0,05 bis 0,5, besonders bevorzugt W:B = 0,1 bis 0,3

Die Kolonne K1 kann aus verschiedenen Materialien gefertigt werden, sofern die verwendeten Materialien gegenüber den eingesetzten Gemischen bei gegebener Temperatur inert sind. Geeignete Materialien sind insbesondere austenische Edelstähle wie 1.4541 oder 1.4571. Auch höherlegierte Werkstoffe, wie der ferritisch/austenitische 1.4462 sind geeignet. Vorzugsweise wird der Werkstoff 1.4541 verwendet.

Am Kopf der Kolonne K1 wird ein Strom O erhalten, der aus dem aufgereinigten Gemisch enthaltend 4,4'-Methylendiphenydiisocyanat besteht und einen Gehalt an hydrolysierbarem Chlor nach ASTM D4663-10 von höchstens 100 ppm, vorzugsweise höchstens 50 ppm, aufweist. Anschließend wird der Strom O einer Abkühlung unterzogen, indem der Strom O auf eine Temperatur von vorzugsweise 10 bis 100°C, insbesondere 20 bis 80 °C, insbesondere 20 bis 60 °C abgekühlt wird.

Die Abkühlung des Stromes O wird vorzugsweise in höchstens 5 Sekunden von der Temperatur am Kopf der Kolonne K1 auf eine Temperatur im Bereich von 20°C bis 60°C, vorzugsweise von 30°C bis 50°C vorgenommen. Durch die schnelle Abkühlung wird die Bildung von Folgeprodukten, insbesondere die Bildung dimerer Folgeprodukte weiter reduziert. Solche Folgeprodukte sind unerwünscht und verringern die Lagerfähigkeit des Produktes.

Entsprechende Verfahren zur schnellen Abkühlung von MDI-haltigen zunächst gasförmigen Strömen ("Quenchen") sind dem Fachmann an sich bekannt. Grundsätzlich kommen direktes Quenchen und indirektes Quenchen in Betracht. Beim direkten Quenchen wird der Strom O in eine flüssige Verbindung A* eingeleitet, welche eine entsprechend niedrige Temperatur aufweist. In einer bevorzugten ersten Ausführungsform ist die Verbindung A* ein Isocyanat, bevorzugt MDI, insbesondere zweikerniges MDI. Besonders bevorzugt ist die Verbindung A* 4,4'-MDI, insbesondere solches, welches das erfindungsgemäße Verfahren bereits durchlaufen hat. In einer zweiten bevorzugten Ausführungsform ist die Verbindung A* ein inertes Lösungsmittel, welches anschließend entfernt werden muss. Entsprechende Verfahren sind dem Fachmann bekannt.

Die Zuführung des gasförmigen Stromes O in die Vorrichtung zum direkten beziehungsweise indirektem Quenchen stellt der Fachmann so ein, dass die erfindungsgemäße Abkühlung in höchstens 5 Sekunden erfolgt, vorzugsweise in höchstens 4 Sekunden, insbesondere in höchstens 3 Sekunden.

Beim indirekten Quenchen erfolgt das Quenchen in einem Wärmetauscher, in dem eine die Wärme aufnehmende Flüssigkeit vorzugsweise im Gegenstrom zum zunächst gasförmigen Strom O geführt wird, ohne in direkten Kontakt mit dem Strom O zu kommen. Durch die Abkühlung kondensiert der Strom O. Der Wärmetauscher wird deshalb auch als Kondensator bezeichnet. Geeignete Wärmetauscher sind dem Fachmann an sich ebenfalls bekannt. Bevorzugte Wärmetauscher sind insbesondere stehende Kondensatoren, Rohrbündelkondensatoren, stehende Rohrbündelkondensatoren und stehende Plattenkondensatoren. Der Begriff stehend kennzeichnet die vertikale Anordnung. Das indirekte Quenchen ist im Rahmen der vorliegenden Erfindung besonders bevorzugt.

In einer besonders bevorzugten Ausführungsform wird dem erfindungsgemäß abzukühlenden gasförmigen Strom O vor dem Quenchen ein inertes gasförmiges Medium, insbesondere Stickstoff, zugemischt.

Vorzugsweise erfolgt die Zumischung des inerten gasförmigen Mediums im Sumpf eines Kondensators (Wärmetauschers), in dem das Quenchen durchgeführt wird. Die Zumischung erfolgt dabei bevorzugt im Gegenstrom durch den Kondensator.

Alternativ erfolgt die Zumischung vor der Zuführung in die Vorrichtung zum Quenchen des gasförmigen Stromes O mittels einer geeigneten Mischvorrichtung oder im Bereich der Zuführung des gasförmigen Stromes O in die Vorrichtung zum Quenchen.

Durch die Mischung mit dem Inertmedium wird der Gehalt an unerwünschten Leichtsiedern im erfindungsgemäßen Verfahren weiter verringert.

Durch die Kombination der Maßnahmen der erfindungsgemäßen destillativen Aufreinigung einerseits und des erfindungsgemäßen Quenchen andererseits werden 4,4'-MDI enthaltende Mischungen erhalten, die sowohl in Bezug auf hydrolysierbare Chlorverbindungen als auch in Bezug auf Dimere eine hohe Reinheit aufweisen.

Erfindungsgemäß enthält das gewonnene Gemisch enthaltend 4,4'-Methylendiphenydiisocyanat noch höchstens 100 ppm an hydrolysierbarem Chlor nach ASTM D4663-10. Vorzugsweise beträgt der Gehalt an hydrolysierbarem Chlor nach ASTM D4663-10 im Strom O höchstens 80 ppm, insbesondere höchstens 50 ppm, besonders bevorzugt höchstens 30 ppm, insbesondere höchstens 20 ppm, ganz besonders bevorzugt höchstens 10 ppm.

Vorzugsweise weist das gewonnene Gemisch enthaltend 4,4'-Methylendiphenydiisocyanat einen Gesamtchlorgehalt nach ASTM D4661-09 von höchstens 200 ppm auf. Besonders bevorzugt beträgt der Gesamtchlorgehalt nach ASTM D4661-09 im Strom O höchstens 150 ppm, insbesondere höchstens 100 ppm, besonders bevorzugt höchstens 70 ppm, insbesondere höchstens 40 ppm, ganz besonders bevorzugt höchstens 20 ppm. Der Gesamtchlorgehalt nach ASTM D4661-09 unterscheidet sich vom Gehalt an hydrolysierbarem Chlor nach ASTM D4663-10 im Wesentlichen durch die Berücksichtigung von kernchlorierten aromatischen Chlorverbindungen.

Vorzugsweise weist das gewonnene Gemisch enthaltend 4,4'-Methylendiphenydiisocyanat eine Acidität nach ASTM D5629-05 von höchstens 40 ppm auf. Besonders bevorzugt beträgt die Acidität nach ASTM D5629-05 höchstens 30 ppm, insbesondere höchstens 20 ppm, ganz besonders bevorzugt höchstens 10 ppm, insbesondere höchstens 5 ppm.

Der Gehalt des aufzureinigenden Gemisches I enthaltend 4,4'-Methylendiphenyldiisocyanat an hydrolysierbarem Chlor gemäß ASTM D4663-10 beträgt erfindungsgemäß mindestens 100 ppm, vorzugsweise mindestens 120 ppm, insbesondere mindestens 150 ppm, besonders bevorzugt mindestens 200 ppm, insbesondere mindestens 250 ppm.

Es ist für den Fachmann offensichtlich, dass durch das erfindungsgemäße Verfahren auch der Gehalt an entsprechenden Bromverbindungen reduziert wird, auch wenn die Charakterisierung des Erfolges über die Chlorverbindungen durchgeführt wird.

Grundsätzlich kommen als aromatische Chlorverbindungen eine Vielzahl von Verbindungen in Betracht, die aus nicht abgetrennten Nebenprodukten der Anilin/Formaldehyd-Kondensation in Folgeprozessen oder anderweitig während der Phosgenierung entstehen.

Oben genannte Reduzierung im Gesamtchlorgehalt nach ASTM D4661-09 betrifft insbesondere halogenierte Phenylisocyanate der allgemeinen Formel I. Unter halogenierten Phenylisocyanaten wird ein mono- oder di- kernchloriertes oder bromiertes Phenylisocyanat verstanden, insbesondere einfach kernchloriertes Phenylisocyanat. Bevorzugt sollte der Gehalt an halogenierten Phenylisocyanaten im Strom O höchstens 25 ppm betragen, besonders bevorzugt höchstens 15 ppm, insbesondere höchstens 10 ppm:

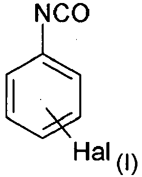

Weitere aromatische Halogenverbindungen, die im aufgereinigten 4,4'-Methylendiphenydiisocyanat in höheren Mengen unerwünscht sind, sind N,N-disubstituierten (sekundäre) Carbaminsäurechloride der allgemeinen Formel II, deren Gehalt im Strom O vorzugsweise höchstens 25 ppm betragen sollte, besonders bevorzugt höchstens 15 ppm, insbesondere höchstens 10 ppm:

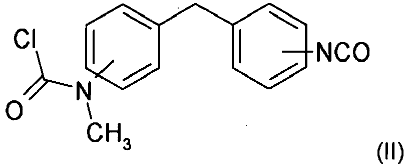

Hierunter fallen insbesondere folgende Verbindungen, die bei der Phosgenierung von Roh-MDA entstehen: 4-Isocyanato-4'-N-methylcarbaminsäurechloridodiphenylmethan, 2-Isocyanato-4'-N-methylcarbaminsäurechloridodiphenylmethan und 2-Isocyanato-2'-N-methylcarbaminsäurechloridodiphenylmethan.

N-Phenyl-N-isocyanatobenzylcarbaminsäurechloride der allgemeinen Formel III, insbesondere N-Phenyl,N-4-isocyanatobenzylcarbaminsäurechlorid und N-Phenyl,N-2-isocyanatobenzylcarbaminsäurechlorid, die bei Phosgenierung aus nicht umgelagerten Aminobenzylanilinen entstehen, fallen ebenfalls in die Klasse der sekundären Carbaminsäurechloride: 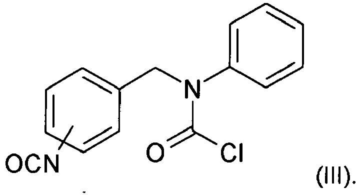 Weitere aromatische Halogenverbindungen, die im aufgereinigten 4,4'-Methylendiphenydiisocyanat in höheren Mengen unerwünscht sind, sind Isocyanatobenzylhalogenide der allgemeinen Formel IV, insbesondere 4-Isocyanatobenzylchlorid und 2-Isocyanatobenzylchlorid, deren Gehalt im Strom O vorzugsweise höchstens 25 ppm betragen sollte, besonders bevorzugt höchstens 15 ppm, insbesondere höchstens 10 ppm:

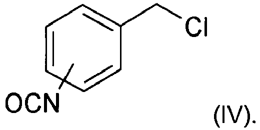

Der Gehalt der vorgenannten Verbindungen der allgemeinen Formel I, II, III und IV wird durch das erfindungsgemäße Verfahren verringert. Vorzugsweise ist der Gehalt an aromatischen Halogenverbindungen der allgemeinen Formel I, II, III und IV im Strom O insgesamt mindestens 10 ppm, vorzugsweise mindestens 50 ppm, besonders bevorzugt mindestens 100 ppm, insbesondere mindestens 150 ppm geringer ist als im Gemisch I. Vorzugsweise beträgt der Gehalt an aromatischen Halogenverbindungen der allgemeinen Formel I, II, III und IV im Strom O insgesamt höchstens 40 ppm, besonders bevorzugt höchstens 30 ppm, ganz besonders bevorzugt höchstens 20 ppm, insbesondere höchstens 10 ppm.

Wie bereits einleitend ausgeführt, kann das erfindungsgemäße Verfahren vorteilhaft in bekannte Verfahren zur Herstellung von Gemischen enthaltend 4,4'- Methylendiphenyldiisocyanat integriert werden, wobei die Verknüpfung mit einer destillativen Auftrennung der Isomere des zweikernigen Methylendiphenyldiisocyanats besonders bevorzugt ist. Ganz besonders bevorzugt ist die Kombination mit einer destillativen Abtrennung von 4,4'-Methylendiphenyldiisocyanat einerseits und ein Gemisch enthaltend 2,4'- und 4,4'-Methylendiphenyldiisocyanat andererseits.

Ein Beispiel für eine destillative Abtrennung von 4,4'-Diisocyanatodiphenylmethan, die mit dem erfindungsgemäßen Verfahren vorteilhaft kombiniert werden kann, ist in der DE-OS 2 631 168 beschrieben, deren Inhalt hiermit in vollem Umfang einbezogen wird. Die DE-OS 2 631 168 beschreibt die mehrstufige Aufarbeitung eines Gemisches aus Polyisocyanatopolyphenylpolymethanen zu Diisocyanatodiphenylmethan-Isomeren. Nach einer destillativen Abtrennung der höherfunktionellen Isocyanate wird der auf dieser Stufe anfallende erste Destillationsstrom, bestehend im Wesentlichen aus 2,2'-MDI, 2,4'-MDI und 4,4'-MDI einer ersten Kolonne zugeführt und in einen weiteren Destillationsstrom und einen Sumpfstrom aufgetrennt. Der Sumpfstrom kann bis zu 10 Gew.-% des ersten Destillationsstroms betragen. Der zweite Destillationsstrom wird in einer zweiten Kolonne in einen Kopfstrom, welcher leichtflüchtige Verunreinigungen, 2,2'-Diisocyanatodiphenylmethan und 2,4'-MDI enthält, und einen Sumpfstrom, welcher die überwiegenden Anteile an 2,4'-MDI und 4,4'-Diisocyanatodiphenylmethan enthält, fraktioniert. Dieser Sumpfstrom wird in einer dritten Kolonne in 4,4'-MDI und einen Destillatanteil, angereichert mit 2,4'-MDI, aufgetrennt. In der letzten Destillationsstufe destilliert 4,4'-MDI mit einem Gehalt von weniger als 2 Gew.-% 2,4'-MDI.

Weitere Verfahren zur destillativen Gewinnung von 4,4'-MDI bzw. von Gemischen aus 4,4'- und 2,4'-MDI sind z.B. in DE-A-2 933 601 und DE-A-3 145 010 beschrieben. In DE-A-3 145 010 wird vorgeschlagen, dass aus dem Isomerengemisch der Diisocyanatodiphenylmethane zunächst als Kopfprodukt 2,2'- und 2,4'-Diisocyanatodiphenylmethan abgezogen werden und als Sumpfprodukt weitgehend von Isomeren befreites 4,4'-MDI erhalten wird. Dieses Sumpfprodukt ist dann in einer Enddestillation von Polymerisationsprodukten, die sich während der thermischen Belastung gebildet haben, zu befreien, während das Kopfprodukt der weiteren destillativen Aufarbeitung gemäß der vorliegenden Erfindung zugeführt werden kann.

In einer bevorzugten Ausführungsform entstammt entsprechend das Gemisch I, welches erfindungsgemäß der Kolonne K1 zugeführt wird, einer zweite Kolonne K2, in der rohes zweikerniges Methylendiphenyldiisocyanat ganz oder teilweise in seine Isomeren getrennt wird, vorzugsweise indem 4,4'-MDI von den Isomeren 2,4'-MDI und/oder 2,2'-MDI ganz oder überwiegen abgetrennt wird, wobei der Gehalt an 4,4'-MDI der abgetrennten Mischung vorzugsweise mindestens 90 Gew.-% 4,4'-MDI bezogen auf das Gesamtgewicht der Mischung beträgt, besonders bevorzugt mindestens 95 Gew.-%, insbesondere mindestens 98 Gew.-%.

Die Kolonne K2 umfasst vorzugsweise Trennelemente, wobei Packungen besonders geeignet sind. Verwendbar sind grundsätzlich aber auch Füllkörper oder Böden. Die Kolonne K2 ist vorzugsweise eine Seitenkolonne. Unter Seitenkolonne ist eine Kolonne zu verstehen, die mindestens einen Sumpfabzug, mindestens einen Seitenabzug und mindestens einen Kopfabzug aufweist.

Je nach Gemischzusammensetzung beträgt die Kopftemperatur der Kolonne K2 bevorzugt 165 bis 200 °C. Der Sumpfdruck beträgt vorzugsweise von 11 bis 20 mbar bei bevorzugten Temperaturen von 210 bis 225 °C. Die Kolonne K2 operiert vorzugsweise bei einem Sumpfdruck von 0,1 bis 50 mbar, bevorzugt von 1 bis 30 mbar, besonders bevorzugt 2 bis 15 mbar und bei einer Sumpftemperatur von 150 bis 250 °C, besonders bevorzugt von 180 bis 240 °C, insbesondere von 200 bis 225°C. Hierdurch wird eine hohe Trennwirkung bei gleichzeitig geringer thermischer Belastung erzielt.

Bei der Destillation des Gemisches isomerer Methylendiphenyldiisocyanate in der Kolonne K2 wird vorzugsweise als Seitenstrom 4,4'-Methylendiphenyldiisocyanat mit einer Isomerenreinheit, d.h. einer Reinheit bezogen auf die drei Isomere 2,2'-MDI, 2,4'-MDI und 4,4'-MDI, von mindestens 97 Gew.-% entnommen (nachfolgend erster Seitenstrom bzw. erster Seitenabzug).

Außerdem wird bei der Destillation des Gemisches isomerer Methylendiphenyldiisocyanate in der Kolonne K2 als zweiter Seitenabzug, der sich oberhalb des ersten Seitenabzuges befindet, oder als Kopfstrom ein Gemisch aus 2,4'-Diisocyanatodiphenylmethan und 4,4'-Diisocyanatodiphenylmethan mit einem Gewichtsverhältnis von 85:15 bis 15:85 erhalten. Die Ausführung mit einem zweiten Seitenabzug oberhalb des ersten Seitenabzuges ist bevorzugt, da hierdurch eine hohe Reinheit an den gewünschten zweikernigen Isomeren erhalten wird. Der Kopfstrom der Kolonne K2 umfasst außerdem die mit dem Feed zugeführten Leichtsiederkomponenten, wie z.B. Monochlorbenzol.

Der Strom, der am zweiten Seitenabzug beziehungsweise am Kopf der Kolonne K2 erhalten wird, weist bevorzugt einen Gehalt an 2,4'-MDI von 20 bis 95 Gew.-% und einen Gehalt an 4,4'-MDI von 5 bis 80 Gew.-%, jeweils bezogen auf das Gesamtgewicht der zweikernigen Isomere von Methylendiphenyldiisocyanat, welches 100 Gew.-% ergibt, auf.

Das Kolonnen-Rücklaufverhältnis (Verhältnis zurücklaufender Stoffstrom zu entnommenem Stoffstrom) im Kopf der Kolonne K2 wird insbesondere im Bereich von 5 bis 250 eingestellt, liegt jedoch besonders bevorzugt im Bereich von 10 bis 120, wobei der Destillatstrom 1 bis 5 Gew.-% bezogen auf den Feedstrom beträgt. Der Sumpfstrom beträgt 60 bis 90 Gew.-%, vorzugsweise 75 bis 85 Gew.-% des Feedstromes.

In einer ersten bevorzugten Ausführungsform ist die Kolonne K2 eine Trennwandkolonne. Der Aufbau einer solchen Kolonne K2 ist dem Fachmann an sich bekannt und wird beispielsweise in der EP 1475367 A1 beschrieben. Die Trennwandkolonne wird vorzugsweise unter den vorstehend für die Kolonne K2 dargelegten Bedingungen betrieben. Die Mischung isomerer zweikerniger Methylendiphenyldiisocyanate wird der Trennwandkolonne vorzugsweise seitlich im Bereich der Trennwand zugeführt. Der Bereich der Trennwand befindet sich im mittleren Bereich der Kolonne K2. Die Länge der Trennwand wird abhängig von den Prozessbedingungen und von den Eigenschaften der eingesetzten Stoffaustauschelemente gewählt. Die Trennwand teilt die Kolonne in eine Vorfraktionierungszone und eine Hauptfraktionierungszone. Als Trennelemente sind Packungen besonders geeignet. Verwendbar sind grundsätzlich aber auch Füllkörper oder Böden.

Alternativ kann die destillative Abtrennung der zweikernigen MDI-Isomere auch zweistufig ausgestaltet sein, wobei eine erste Destillationsstufe in einer Destillationskolonne ohne Trennwand, und eine zweite Stufe mit Trennwandkolonne durchgeführt wird oder wobei zwei Trennwandkolonnen Verwendung finden. Entsprechende Verfahren werden in der EP 1475367 A1 in den Absätzen [0024] bis [0031] ausgeführt.

In einer zweiten bevorzugten Ausführungsform ist die Kolonne K2 eine Seitenkolonne ohne Trennwand. Die bevorzugten Parameter der Seitenkolonne wurden bereits vorstehend beschrieben. Dabei werden mittels des Kopfabzuges vorzugsweise 2,2'-MDI und Leichtsieder und mittels des Sumpfabzuges vorzugsweise 4,4'-MDI und Hochsieder abgetrennt, wobei der Strom aus Sumpf- und Kopfabzug wieder in Schritt (b) des erfindungsgemäßen Verfahrens eingesetzt werden kann. Der Seitenkolonne wird vorzugsweise wie oben beschrieben in einem ersten Seitenabzug 4,4'-MDI in einer Reinheit von mindestens 97 Gew.-% bezogen auf das Gesamtgewicht des Stoffstromes entnommen sowie oberhalb des ersten Seitenabzuges in einem zweiten Seitenabzug das oben beschriebene Gemisch aus 4,4'-MDI und 2,4'-MDI.

Das Gemisch I wird der Kolonne K2 bevorzugt als Seitenstrom entnommen und in gasförmiger Form der Kolonne K1 zugeführt. Das Gemisch I ist 4,4'-MDI mit vorzugsweise mindestens 98 Gew.-%, besonders bevorzugt 98,5 bis 99,0 Gew.-% Reinheit bezogen auf das Gesamtgewicht des Gemisches I.

Bevorzugt wird der gasförmige Strom bestehend aus dem Gemisch I in der Kolonne K1 mit mindestens einer flüssigen Verbindung A in Kontakt gebracht, welche erfindungsgemäß den gleichen oder einen höheren Siedepunkt als 4,4'-Methylendiphenyldiisocyanat aufweist und welche erfindungsgemäß einen Gehalt an hydrolysierbarem Chlor nach ASTM D4663-10 von höchstens 1000 ppm aufweist.

In einer bevorzugten Ausführungsform wird das im Sumpf der Kolonne K1 erhaltene Produkt in die zweite Kolonne K2 zurückgeführt. Hierdurch kann einerseits die Bildung von Abfallprodukten vermieden werden, so dass die Ausbeute des Gesamtverfahrens optimiert wird. Andererseits wird gewährleistet, dass gleichzeitig besonders reines 4,4'-MDI erhalten wird.

Der Strom, der am Kopf der Kolonne K2 erhalten wird, weist bevorzugt einen Gehalt an 2,4'-MDI von 20 bis 95 Gew.-% und einen Gehalt an 4,4'-MDI von 5 bis 80 Gew.-%, jeweils bezogen auf das Gesamtgewicht der zweikernigen Isomere von Methylendiphenyldiisocyanat, welches 100 Gew.-% ergibt, auf. Außerdem enthält der Kopfstrom der Kolonne K2 die mit dem Feed zugeführten Leichtsiederkomponenten wie z.B. Chlorbenzol.

Die erfindungsgemäß hergestellten Mischungen aus Diphenylmethandiisocyanat-Isomeren, insbesondere aus 2,4'- und 4,4'-Diphenylmethan-diisocyanaten eignen sich vorzüglich zur Herstellung von Polyurethanklebstoffen und -überzügen. Reines 4,4'-Diphenylmethan-diisocyanat wird vorzugsweise zur Herstellung von Polyurethan-Elastomeren, -Fäden und -Borsten verwendet. Aufgrund des geringen Gehaltes an hydrolysierbaren Chlorverbindungen sind die Polyurethane relativ stabil gegen Vergilben unter dem Einfluß von Luft und Licht.

Eine weitere Aufgabe bestand darin, die Reaktivität des MDIs zu stabilisieren. Im Rahmen des Betriebs wurde gefunden, dass Reaktiviätsschwankungen im MDI verringert werden, indem heiße Isocyanatleitungen mit einer Temperatur von > 120° zwischen Anlagenteilen aus korrosionsfesteren Materialien (Nickel-Basislegierungen) gebaut werden. Besonders trifft dies auf Leitungen mit einer Temperatur zwischen 180°C und 220°C zu.

Zwischen Anlagenteilen meint beispielsweise Transportleitungen zwischen zwei benachbarten Anlagensektionen, Produkttransport zwischen zwei Kolonnen, oder den Transport vom Kolonnenablauf zum Produktkühler in den Produkttank. In diesen Leitungen beträgt der Isocyanatgehalt mehr als 20w% gemessen nach DIN ISO 14896:2009.

Geeignete Materialien dafür sind Hastelloy oder Inconel Legierungen. Der Nickelgehalt diese Legierungen beträgt dabei mindestens 50w%. Geeignete Werkstoffe für diese Leitungen sind also beispielsweise Alloy 200 (2.4066), Alloy 400 (2.4360), Alloy C-276 (2.4819), Alloy C-22 (2.4602), Alloy 59 (2.4605), Hastelloy C-4 (2.4610), Hastelloy C-22 (2.4602), sowie alle gängigen Hastelloy Subgruppen.

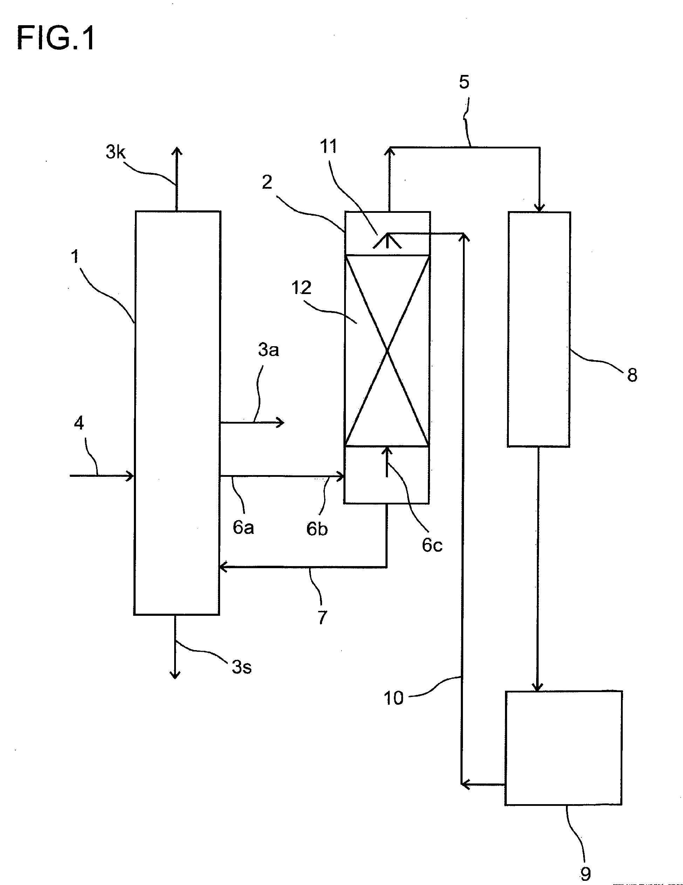

Eine bevorzugte Ausführungsform der vorliegenden Erfindung ist in Fig. 1 schematisch veranschaulicht. Fig. 1 dient dabei der Erläuterung dieser bevorzugten Ausführungsform der vorliegenden Erfindung und ist nicht einschränkend zu verstehen. Einzelne Elemente der nachfolgend erläuterten bevorzugten Ausführungsform können vorteilhaft mit oben erläuterten Ausführungsformen kombiniert werden.
1 - Kolonne K2
2 - Kolonne K1
3a - oberer Seitenabzug der Kolonne K2
3k - Kopfstrom aus der Kolonne K2
3s - Sumpfstrom aus der Kolonne K2
4 - Strom 2-kernige MDI-Isomere (feed Kolonne K2)
5 - Kopfstrom O der Kolonne K1
6a - unterer Seitenabzug der Kolonne K2
6b - Strom aus Gemisch I
6c - Dampf aus Gemisch I
7 - Sumpfstrom aus Kolonne K1
8 - Kondensator
9 - Lagertank für flüssiges 4,4'-MDI
10 - Rückführung aus Lagertank
11 - Flüssigkeitsverteiler
12 - Packungselemente

Beschreibung der bevorzugten Ausführungsform gemäß Fig. 1:
Ein Strom (4) enthaltend eine Mischung zweikerniger Isomere des MDI wird der Kolonne K2 (1) zugeführt. Am Sumpf der Kolonne K2 (1) wird der Sumpfstrom (3s) erhalten und am Kopf der Kopfstrom (3k). Aus einem ersten Seitenabzug (6a) wird ein Strom (6b) entnommen, welcher bevorzugt mindestens 98 Gew.-% 4,4'-MDI enthält sowie oberhalb des ersten Seitenstromes ein Strom (3a), welcher aus 4,4'-MDI und 2,4'-MDI besteht. Der Strom (6b) bildet den Strom I, welcher der Kolonne K1 (2) in gasförmigem Zustand zugeführt wird und als Dampf bestehend aus dem Gemisch I (6c) die Packungselemente (12) der Kolonne K1 (2) durchläuft. Im Gegenstrom zur Richtung des Stromes 6c durchläuft 4,4'-MDI, welches einem Lagertank (9) für flüssiges 4,4'-MDI entnommen und über eine Rückführung (10) am Kopf der Kolonne K1 einem Flüssigkeitsverteiler (11) zugeführt wird. Der Flüssigkeitsverteiler (11) sorgt für eine hohe Kontaktfläche zwischen dem aufsteigenden gasförmigen Strom 6c und dem sich im Gegenstrom bewegenden flüssigen Strom aus 4,4'-MDI. Am Sumpf der Kolonne K1 (2) wird der Strom (7) erhalten, welcher in die Kolonne K2 (1) zurückgeführt wird. Am Kopf der Kolonne K1 wird der Kopfstrom O (5) erhalten, welcher aus dem aufgereinigten 4,4'-MDI besteht. Der gasförmige Strom (5) wird einem Kondensator (8) zugeführt, in welchem der Strom (5) kondensiert wird. Anschließend wird das so erhaltene hochreine flüssige 4,4'-MDI einem Lagertank (9) zugeführt.

### Beispiel 1 (erfindungsgemäß)

Es wurde eine Apparatur verwendet, wie sie in Fig. 1 schematisch dargestellt ist. Die Isocyanatleitungen zur Kolonne K2 (Strom 4), sowie die Ströme Strom 3s, 7 waren aus Hastelloy-C4 gefertigt.

2,0 kg/h Roh-MDI enthaltend 50,2 Gew.-% 4,4'-MDI, 6,8 Gew.-% 2,4'-MDI, 21,2 Gew.-% 3-Ring MDI wurden in einem Fallfilmverdampfer aus dem Werkstoff 1.4571 bei einem Druck von 5 mbar verdampft.

Nach der Kondensation wurde ein Destillat enthaltend 85,1 Gew.-% 4,4'-MDI, 12,6 Gew.-% 2,4'-MDI, 2,3 Gew.-% 3-Ring-MDI gewonnen. Der Massenstrom des am Kondensator niedergeschlagenen Destillats betrug 0,690 kg/h.

Das Destillat wurde einer Seitenabzugskolonne K2 in flüssiger Form zugeführt. Die Kolonne K2 und der Sumpfverdampfer bestanden aus dem Werkstoff 1.4571. Die Kolonne war mit strukturierten Packungen ausgestattet, die einen geringen Druckverlust aufwiesen Der Kopfdruck der Kolonne betrug 5 mbar.

Die Kolonne K2 bestand aus einem Verstärkungs- und einem Abtriebsteil. Im Abtriebsteil der Kolonne K2 wurde der Strom I gasförmig mittels eines ersten Seitenabzuges (6a) unterhalb eines Packungselements entnommen und der Kolonne K1 zugeführt (Strom aus Gemisch I). Der Sumpfablauf der Kolonne K1 (7) wurde der Kolonne K2 unterhalb des Zulaufs (Feed) zur Kolonne K2 zugeführt. Oberhalb des Zulaufs wurde an einem oberen Seitenabzug (3a) eine flüssige Fraktion entnommen.

Der Strom I (6b) enthielt 98,7 Gew.-% 4,4'-MDI und 1,3 Gew.-% 2,4'-MDI. Der Gehalt an 3-Ring Verbindungen betrug 530 ppm. Der Massenstrom (6) betrug 0,79 kg/h. Am Strom (3k) wurde eine Mischung von 47 Gew.-% 4,4'-MDI und 53 Gew.-% 2,4'-MDI abgezogen. Der Massenstrom betrug 0,14 kg/h.

Aufreinigung Seitenstrom (6): Der gasförmige Seitenstrom (Dampf aus Gemisch I, 6c) wurde der Kolonne K1 (2) zugeführt. Die Kolonne K1 aus dem Werkstoff 1.4571 war mit einer druckverlustarmen Packung (strukturiert) mit einer spezifischen Oberfläche von 500 m²/m³ ausgestattet. Der Kopfdruck der Kolonne betrug 15 mbar.

Zuführung der flüssigen Verbindung A: Am Kopf der Kolonne K1 wurde ein Strom der Verbindung A bestehend aus 98,5 Gew.-% 4,4'-MDI und 1,5 Gew.-% 2,4'-MDI in flüssiger Form oberhalb des höchsten Packungselements zugegeben und mittels eines Kastenrinnen-Flüssigkeitsverteilers (11) verteilt. Der Massenstrom der Verbindung A betrug 0,17 kg/h. Die Flüssigkeit A bestand aus bei 42°C gelagertem 4,4'-MDI aus einem Lagertank (9), in welchem hochreines 4,4'-MDI aufgenommen wurde, welches das Verfahren schon durchlaufen hat. Der Dimergehalt der Verbindung A betrug 0,13 Gew.-%. Das Gewichtsverhältnis von in Kolonne K1 zurückgeführtem Produkt (10) zu der dem Lagertank (9) zugeführtem Produkt betrug kontinuierlich 0,26 g/g.

Am Kopf der Kolonne K1 wurde als Produkt ein gasförmiger Strom O (5) erhalten, der aus 98,5 Gew.-% 4,4'-MDI und 1,5 Gew.-% 2,4'-MDI bestand. Der Massenstrom betrug hier 0,65 kg/h. Der Strom O (5) wurde binnen 5s in einem Kondensator (8) auf 42°C abgekühlt und dem Lagertank (9) zugeführt.

Im gasförmigen Strom O war der Gehalt an hydrolysierbarem Chlor nach ASTM D4663-10 im erfindungsgemäßen Bereich und konnte gegenüber dem Gehalt im Strom I signifikant reduziert werden.

Mit oben genannten Proben wurden Gaschromatographie (GC)-Untersuchungen durchgeführt. Die Zuordnung der Komponenten erfolgte durch Gegenüberstellung von Aufnahmen mit Flammenionisationsdetektor (FID) und Elektroneneinfangdetektor (ECD) sowie durch Untersuchungen mittels Kopplung von GC mit Massenspektrometrie (GC/MS). N,N-disubstituierte Carbaminsäurechloride der Formel II waren nicht nachweisbar. Der Gehalt an kernchloriertem MDI betrug 0,020 Flächen-% (Fläche der genannten Verbindung bezogen auf die ausgewertete Gesamtfläche im Chromatogramm).

Gleichzeitig wurde ein Gemisch mit geringem Gesamtchlorgehalt nach ASTM D4661-09 und mit geringer Acidität nach ASTM D5629-05 erhalten.

### Beispiel 2 (nicht erfindungsgemäß)

Die Durchführung erfolgte wie in Beispiel 1, nur dass am Kopf der Kolonne K1 der Massenstrom der Komponente A auf 0 kg/h reduziert wurde. Von oben genannten Proben wurden analog Beispiel 1 GC-Untersuchungen durchgeführt. N,N-disubstituierte Carbaminsäurechloride der Formel II wurden zu 0,00047 Flächen-% bestimmt. Der Gehalt an kernchloriertem MDI betrug 0,024 Flächen-%.

## Patentansprüche

1. Verfahren zur Aufreinigung von 4,4'-Methylendiphenyldiisocyanat enthaltenden Mischungen umfassend die destillative Aufreinigung eines Gemisches I enthaltend 4,4'-Methylendiphenyldiisocyanat mit einem Gehalt an hydrolysierbarem Chlor nach ASTM D4663-10 von mehr als 100 ppm mittels einer Kolonne K1, wobei der gasförmige Strom bestehend aus dem Gemisch I in der Kolonne K1 mit mindestens einer flüssigen Verbindung A in Kontakt gebracht wird, welche den gleichen oder einen höheren Siedepunkt als 4,4'-Methylendiphenyldiisocyanat aufweist und welche einen Gehalt an hydrolysierbarem Chlor nach ASTM D4663-10 von höchstens 100 ppm aufweist, und wobei der am Kopf der Kolonne erhaltene gasförmige Strom O enthaltend 4,4'-Methylendiphenyldiisocyanat einen Gehalt an hydrolysierbarem Chlor nach ASTM D4663-10 von höchstens 100 ppm aufweist.

2. Verfahren nach Anspruch 1, wobei die Verbindung A 4,4'-Methylendiphenyldiisocyanat ist, welches die destillative Aufreinigung gemäß Anspruch 1 bereits durchlaufen hat.

3. Verfahren nach Anspruch 1 oder 2, wobei die Verbindung A 4,4'-Methylendiphenyldiisocyanat ist, welches aus einer Vorrichtung zur Lagerung von 4,4'-Methylendiphenyldiisocyanat zurückgeführt wird.

4. Verfahren nach Anspruch 1, wobei die Verbindung A eine gegenüber 4,4'-Methylendiphenyldiisocyanat inerte Verbindung ist.

5. Verfahren nach einem oder mehreren der Ansprüche 1 bis 4, wobei die Zugabe der flüssigen Verbindung A oberhalb des obersten Trennelementes der Kolonne K1 erfolgt.

6. Verfahren nach einem oder mehreren der Ansprüche 1 bis 5, wobei die Kolonne K1 eine Packungskolonne ist.

7. Verfahren nach einem oder mehreren der Ansprüche 1 bis 6, wobei die Zugabe der flüssigen Verbindung A in die Kolonne K1 durch einen Flüssigkeitsverteiler erfolgt.

8. Verfahren nach einem oder mehreren der Ansprüche 1 bis 7, wobei die Zuführung des Gemisches I in die Kolonne K1 in gasförmiger Form erfolgt.

9. Verfahren nach einem oder mehreren der Ansprüche 1 bis 8, wobei die Abkühlung des Stromes O in höchstens 5 Sekunden auf eine Temperatur von 20°C bis 60°C vorgenommen wird.

10. Verfahren nach einem oder mehreren der Ansprüche 1 bis 9, wobei der Gehalt des Stromes O und der Verbindung A an hydrolysierbarem Chlor nach ASTM D4663-10 jeweils höchstens 50 ppm beträgt.

11. Verfahren nach einem oder mehreren der Ansprüche 1 bis 10, wobei der Gesamtchlorgehalt nach ASTM D4661-09 im Strom O höchstens 100 ppm beträgt.

12. Verfahren nach einem oder mehreren der Ansprüche 1 bis 11, wobei der Gehalt an halogenierten Phenylisocyanaten im Strom O höchstens 25 ppm beträgt.

13. Verfahren nach einem oder mehreren der Ansprüche 1 bis 12, wobei der Gehalt an N,N-disubstituierten Carbaminsäurechloriden im Strom O höchstens 25 ppm beträgt.

14. Verfahren nach einem oder mehreren der Ansprüche 1 bis 13, wobei der Gehalt an hydrolysierbarem Chlor nach ASTM D4663-10 im Strom O mindestens 10 ppm geringer ist als im Gemisch I.

15. Verfahren nach einem oder mehreren der Ansprüche 1 bis 14, wobei das Gemisch I vor Zuführung in die Kolonne K1 einer zweiten Kolonne K2 entnommen wird, in der rohes zweikerniges Methylendiphenyldiisocyanat ganz oder teilweise in seine Isomeren aufgetrennt wird.

## Claims

1. A method for purifying mixtures comprising 4,4'methylenediphenyl diisocyanate, which comprises purifying by distillation a mixture I comprising 4,4'-methylenediphenyl diisocyanate having a hydrolyzable chlorine content as specified in ASTM D4663-10 of greater than 100 ppm by means of a column K1, wherein the gaseous stream comprising the mixture I is brought into contact in the column K1 with at least one liquid compound A which has the same or higher boiling point than 4,4'methylenediphenyl diisocyanate and which has a hydrolyzable chlorine content as specified in ASTM D4663-10 of a maximum of 100 ppm, and wherein the gaseous stream 0 obtained at the top of the column and comprising 4,4'-methylenediphenyl diisocyanate has a hydrolyzable chlorine content as specified in ASTM D4663-10 of a maximum of 100 ppm.

2. The method according to claim 1, wherein the compound A is 4,4'-methylenediphenyl diisocyanate that has already passed through the purification by distillation according to claim 1.

3. The method according to either claim 1 or 2, wherein the compound A is 4,4'-methylenediphenyl diisocyanate which is recirculated from a device for storing 4,4'-methylenediphenyl diisocyanate.

4. The method according to claim 1, wherein the compound A is a compound that is inert to 4,4'methylenediphenyl diisocyanate.

5. The method according to one or more of claims 1 to 4, wherein the liquid compound A is added above the uppermost separation element of the column K1.

6. The method according to one or more of claims 1 to 5, wherein the column K1 is an ordered-packing column.

7. The method according to one or more of claims 1 to 6, wherein the liquid compound A is added to the column K1 by a liquid distributor.

8. The method according to one or more of claims 1 to 7, wherein the mixture I is fed into the column K1 in gaseous form.

9. The method according to one or more of claims 1 to 8, wherein the stream 0 is cooled in at most 5 seconds to a temperature of 20°C to 60°C.

10. The method according to one or more of claims 1 to 9, wherein the hydrolyzable chlorine content of the stream 0 and the compound A as specified in ASTM D4663-10 is in each case a maximum of 50 ppm.

11. The method according to one or more of claims 1 to 10, wherein the total chlorine content as specified in ASTM D4661-09 in stream 0 is a maximum of 100 ppm.

12. The method according to one or more of claims 1 to 11, wherein the content of halogenated phenyl isocyanates in stream 0 is a maximum of 25 ppm.

13. The method according to one or more of claims 1 to 12, wherein the content of N,N-disubstituted carbamoyl chlorides in stream 0 is a maximum of 25 ppm.

14. The method according to one or more of claims 1 to 13, wherein the hydrolyzable chlorine content as specified in ASTM D4663-10 in stream 0 is at least 10 ppm less than in the mixture I.

15. The method according to one or more of claims 1 to 14, wherein the mixture I, before feeding into the column K1, is taken off from a second column K2 in which crude binuclear methylenediphenyl diisocyanate is completely or partially separated into its isomers.

## Revendications

1. Procédé pour la purification de mélanges contenant du diisocyanate de 4,4'-méthylènediphényle comprenant la purification par distillation d'un mélange I contenant du diisocyanate de 4,4'-méthylènediphényle présentant une teneur en chlore hydrolysable selon la norme ASTM D4663-10 supérieure à 100 ppm au moyen d'une colonne K1, le flux gazeux, constitué par le mélange I, étant mis en contact dans la colonne K1 avec au moins un composé liquide A qui présente le même point d'ébullition ou un point d'ébullition supérieur à celui du diisocyanate de 4,4'-méthylènediphényle et qui présente une teneur en chlore hydrolysable selon la norme ASTM D4663-10 d'au plus 100 ppm, et le flux gazeux 0 obtenu en tête de la colonne, contenant du diisocyanate de 4,4'-méthylènediphényle, présentant une teneur en chlore hydrolysable selon la norme ASTM D4663-10 d'au plus 100 ppm.

2. Procédé selon la revendication 1, le composé A étant du diisocyanate de 4,4'-méthylènediphényle, qui a déjà traversé la purification par distillation selon la revendication 1.

3. Procédé selon la revendication 1 ou 2, le composé A étant du diisocyanate de 4,4'-méthylènediphényle qui est recyclé à partir d'un dispositif destiné à l'entreposage de diisocyanate de 4,4'-méthylènediphényle.

4. Procédé selon la revendication 1, le composé A étant un composé inerte par rapport au diisocyanate de 4,4'-méthylènediphényle.

5. Procédé selon l'une ou plusieurs des revendications 1 à 4, l'addition du composé liquide A ayant lieu au-dessus de l'élément de séparation le plus élevé de la colonne K1.

6. Procédé selon l'une quelconque des revendications 1 à 5, la colonne K1 étant une colonne à garnissage.

7. Procédé selon l'une ou plusieurs des revendications 1 à 6, l'addition du composé liquide A dans la colonne K1 ayant lieu par un distributeur de liquide.

8. Procédé selon l'une ou plusieurs des revendications 1 à 7, l'addition du mélange I dans la colonne K1 ayant lieu sous forme gazeuse.

9. Procédé selon l'une ou plusieurs des revendications 1 à 8, le refroidissement du flux 0 étant réalisé en au plus 5 secondes à une température de 20°C jusqu'à 60°C.

10. Procédé selon l'une ou plusieurs des revendications 1 à 9, la teneur du flux 0 et du composé A en chlore hydrolysable selon la norme ASTM D4663-10 étant à chaque fois d'au plus 50 ppm.

11. Procédé selon l'une ou plusieurs des revendications 1 à 10, la teneur totale en chlore selon la norme ASTM D4661-09 dans le flux 0 étant d'au plus 100 ppm.

12. Procédé selon l'une ou plusieurs des revendications 1 à 11, la teneur en isocyanates de phényle halogénés dans le flux 0 étant d'au plus 25 ppm.

13. Procédé selon l'une ou plusieurs des revendications 1 à 12, la teneur en chlorures d'acide carbamique N,N-disubstitués dans le flux 0 étant d'au plus 25 ppm.

14. Procédé selon l'une ou plusieurs des revendications 1 à 13, la teneur en chlore hydrolysable selon la norme ASTM D4663-10 dans le flux 0 étant inférieure d'au moins 10 ppm à celle dans le mélange I.

15. Procédé selon l'une ou plusieurs des revendications 1 à 14, le mélange I étant prélevé, avant l'introduction dans la colonne K1, d'une deuxième colonne K2, dans laquelle le diisocyanate de méthylènediphényle à deux noyaux brut est séparé totalement ou partiellement en ses isomères.
